(19) Europäisches Patentamt
European Patent Office
Office européen des brevets

(11) Numéro de publication: **0 084 993**
**B1**

(12) **FASCICULE DE BREVET EUROPEEN**

(45) Date de publication du fascicule du brevet: **24.07.85**

(21) Numéro de dépôt: **83400091.1**

(22) Date de dépôt: **14.01.83**

(51) Int. Cl.[4]: **C 07 D 405/12**, C 07 D 417/12, A 61 K 31/425, A 61 K 31/495, A 61 K 31/505, C 07 D 413/12

(54) **Nouveaux dérivés de benzodioxine, leurs procédés de préparation et les compositions pharmaceutiques les renfermant.**

(30) Priorité: **21.01.82 FR 8200870**

(43) Date de publication de la demande: **03.08.83 Bulletin 83/31**

(45) Mention de la délivrance du brevet: **24.07.85 Bulletin 85/30**

(84) Etats contractants désignés: **AT BE CH DE FR GB IT LI LU NL SE**

(56) Documents cités:
**FR - M - 4 770**
**FR - M - 8 193**
**US - A - 3 362 956**

(73) Titulaire: **ADIR, 22, rue Garnier, F-92200 Neuilly-sur-Seine (FR)**

(72) Inventeur: **Regnier, Gilbert, Avenue du Plessis, F-92290 Châtenay-Malabry (FR)**
Inventeur: **Poignant, Jean-Claude, Rue de la Fontaine St-Mathieu, F-91440 Bures-sur-Yvette (FR)**

(74) Mandataire: **Reverbori, Marcelle et al, ADIR 22 Rue Garnier, F-92200 Neuilly-sur-Seine (FR)**

**EP 0 084 993 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés de benzodioxine, leurs procédés de préparation et les compositions pharmaceutiques les renfermant.

Elle concerne particulièrement les dérivés de benzodioxine de formule générale:

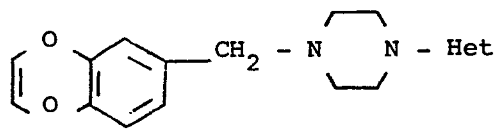

(I)

dans laquelle Het représente un radical pyridyle, pyrimidinyle, pyrazinyle, thiazolyle, thiadiazolyle, oxadiazolyle ou triazolyle éventuellement substitué par un radical méthyle ou méthoxy.

L'état de la technique dans ce domaine est illustré par les brevets N[os] FR M 4770 (Science Union), USA A 3362956 (Sydney Archer) et FR M 8193 (Science Union).

La présente invention concerne également les sels d'addition des dérivés de formule générale I avec des acides, et plus particulièrement les sels d'addition acides qui sont physiologiquement tolérables.

En effet les dérivés de formule générale I sont des bases faibles qui peuvent être transformées avec des acides en sels d'addition acides. Comme acides qui peuvent être utilisés pour la formation de ces sels, on peut citer par exemple, dans la série minérale, les acides chlorhydrique, bromhydrique, sulfurique et phosphorique, et dans la série organique, les acides acétique, propionique, maléique, fumarique, tartrique, citrique, oxalique, benzoïque, méthane sulfonique et iséthionique.

La présente invention a également pour objet le procédé de préparation des dérivés de formule générale I, caractérisé en ce que:

– soit l'on condense un dérivé halogéné de formule générale:

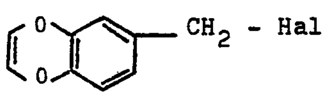

(II)

dans laquelle Hal est un atome de chlore ou de brome, avec une pipérazine N-monosubstituée de formule générale:

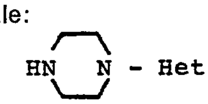

(III)

dans laquelle Het a la signification précédemment définie,

– soit l'on condense un dérivé halogéné de formule générale:

Het-Hal (IV)

dans laquelle Het et Hal ont les significations énoncées prédécemment, avec une pipérazine N-monosubstituée de formule:

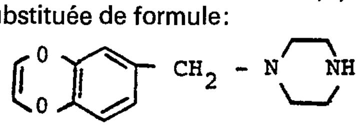

(V)

Dans les deux cas, il est avantageux d'effectuer la condensation en solution dans un solvant polaire tel que par exemple un alcool à haut point d'ébullition comme le butanol ou le pentanol, ou de préférence un amide aliphatique comme par exemple le diméthylformamide ou le diméthylacétamide. Il est avantageux d'opérer à une température comprise entre 110 et 150° C en présence d'un accepteur de l'hydracide formé au cours de la réaction. Comme accepteurs, on peut mentionner, par exemple, les sels alcalins et alcalino-terreux de l'acide carbonique tels que par exemple les bicarbonates et carbonates de sodium et de potassium, le carbonate de calcium, ou les amines tertiaires telles que par exemple le triéthylamine ou la pyridine. Si on le désire, il est également possible de remplacer de tels sels ou bases par un excès de la pipérazine monosubstituée de formule III ou V, cet excès agissant comme accepteur de l'hydracide formé.

La présente invention a également pour objet le procédé de préparation des dérivés de formule générale I, caractérisé en ce que l'on soumet un mélange de l'aldéhyde de formule:

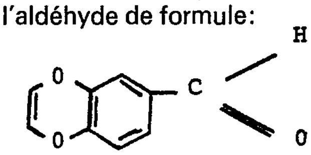

(VI)

et d'une pipérazine N-monosubstituée de formule générale III précédemment définie, à une alkylation réductrice, à l'aide d'un cyanoborohydrure de formule $BH_3$ CN M dans laquelle M représente un métal alcalin tel que par exemple sodium ou potassium.

Cette alkylation réductive est avantageusement conduite selon la méthode décrite par R. Borch et coll., «J. Am. Chem. Soc.», *93*, 2897 (1971), en opérant à température ambiante à l'aide de cyanoborohydrure de sodium, en présence d'acide chlorhydrique ou acétique, à pH 6-8, dans un solvant approprié comme par exemple le tétrahydrofuranne, le dioxanne ou un alcool miscible à l'eau à bas poids moléculaire tel que le méthanol ou l'éthanol.

Les matières premières utilisées pour ces procédés sont des composés connus, ou sont préparées selon des méthodes décrites dans la littérature, pour préparer des composés analogues, comme mentionné dans les exemples suivants.

Les dérivés de formule générale I peuvent être purifiés par des méthodes physiques telles que distillation, cristallisation ou chromatographie, ou par des méthodes chimiques comme, par exemple la formation de sels d'addition, cristallisation de ces derniers et décomposition par les agents alcalins.

Les dérivés de formule générale I et leurs sels physiologiquement tolérables, possèdent des propriétés pharmacologiques et thérapeutiques intéressantes, dont les propriétés psychotropes à mécanisme dopaminergique central. Ils peuvent être utilisés comme médicament notamment dans le traitement de troubles psychotropes liés à un dysfonctionnement des voies dopaminergiques.

Leur toxicité est faible et leur $DL_{50}$ déterminée chez la souris par voie intrapéritonéale est supérieure à 300 mg/kg.

L'activité centrale dopaminergique des produits de l'invention a été déterminée notamment par la

mesure des rotations chez le rat selon la méthode de Ungerstedt U., «European Journal of Pharmacology», 5, (1968) 107-110. Lorsque les composés de l'invention sont administrés à la dose de 25 mg/kg par voie sous-cutanée, on enregistre jusqu'à 335 rotations en 45 min.

La présente invention comprend également les compositions pharmaceutiques contenant comme principe actif un dérivé de formule générale I ou un de ses sels d'addition acides physiologiquement tolérables mélangé ou associé à un excipient pharmaceutique approprié, comme par exemple, l'eau distillée, le glucose, lactose, amidon, talc, éthylcellulose, stéarate de magnésium ou beurre de cacao.

Les compositions pharmaceutiques ainsi obtenues se présentent généralement sous forme dosée et peuvent contenir de 5 à 50 mg de principe actif. Elles peuvent revêtir la forme de comprimés, dragées, gélules, suppositoires, solutions injectables, ou buvables et être administrées par voie orale, rectale ou parentérale à la dose de 5 à 50 mg, 1 à 3 fois par jour.

Les exemples suivants illustrent l'invention, les points de fusion étant déterminés dans un tube capillaire, sauf indication contraire.

*Exemple 1:*

*(Benzodioxinyl-6 méthyl)-2 (pyrimidinyl-2)-4 pipérazine*

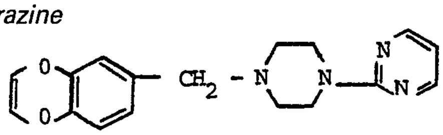

*Première méthode:*

On chauffe pendant 3 h à reflux une solution de 24,5 g (0,134 mol) de chlorométhyl-6 benzodioxine et de 44 g (0,268 mol) de (pyrimidinyl-2)-1 pipérazine dans 500 ml de xylène et 50 ml de diméthylformamide. Lorsque la réaction est terminée on refroidit le mélange et le traite avec 200 ml d'eau. La couche xylénique est décantée et évaporée sous pression réduite. On recueille 37 g d'huile orange que l'on dissout dans 200 ml d'éthanol anhydre. Après addition d'un excès d'éther chlorhydrique, le dichlorhydrate cristallise. On recueille 39 g de dichlorhydrate de (benzodioxinyl-6 méthyl)-1-(pyrimidinyl–2)-4 pipérazine sous forme de cristaux blancs fondant à 125-220° C.

La chlorométhyl-6 benzodioxine de départ a été préparée par chloration dans le benzène au moyen de $SOCl_2$, de l'hydroxyméthyl-6 benzodioxine, p.f. 50° C, elle-même préparée par réduction par $LiAlH_4$ dans le tétrahydrofuranne, de la carbométhoxy-6 benzodioxine, elle-même préparée selon G. Coudert et coll., «Tetrahedron letters», *1978*, 1059-62.

*Deuxième méthode:*

On chauffe pendant 6 h à reflux une solution de 11,4 g (0,1 mol) de chloro-2 pyrimidine et de 23,2 g (0,1 mol) de (benzodioxinyl-6 méthyl)-1 pipérazine (huile) dans 500 ml de pentanol, en présence de 13,8 g (0,1 mol) de carbonate de potassium. Lorsque la réaction est terminée on filtre le sel formé et évapore le solvant sous pression réduite. Le résidu huileux obtenu pesant 30 g est dissous dans 160 ml d'éthanol anhydre. Par addition d'un excès d'éther chlorhydrique, le dichlorhydrate cristallise. On obtient finalement 30 g de dichlorhydrate de (benzodioxinyl-6 méthyl)-1-(pyrimidinyl-2)-4 pipérazine, sous forme de cristaux blancs fondant à 215-220° C.

La (benzodioxinyl-6 méthyl)-1 pipérazine de départ a été préparée par chauffage de la chlorométhyl-6 benzodioxine dans le butanol avec un excès de pipérazine anhydre.

*Troisième méthode:*

A une solution de 8,1 g (0,05 mol) de formyl-6 benzodioxine (préparée selon G. Coudert et coll., «Tetrahedron letters», *1978*, 1059-62), et de 8,2 g (0,05 mol) de (pyrimidinyl-2)-1 pipérazine dans un mélange de 50 ml de tétrahydrofuranne et 50 ml de méthanol, on ajoute 3,1 g (0,05 mol) de cyanoborohydrure de sodium et 10 ml de méthanol chlorhydrique 5N. On maintient l'agitation pendant 2 h à température ambiante puis acidifie à pH 1 avec HCl 2N. On évapore le solvant sous pression réduite, traite le résidu avec 50 ml d'eau, extrait à l'éther et alcalinise avec un excès de carbonate de potassium. On extrait enfin la base au chloroforme. Après un traitement par un excès d'éther chlorhydrique, le dichlorhydrate cristallise. On obtient finalement 7 g de dichlorhydrate de (benzodioxinyl-6 méthyl)-1 (pyrimidinyl-2)-4 pipérazine, sous forme de cristaux blancs fondant à 215-220° C.

*Exemples 2 à 9:*

Les dérivés suivants ont été préparés selon les procédés décrits dans l'exemple 1:

2. Benzodioxinyl-6 méthyl)-1 (pyridyl-2)-4 pipérazine, p.f. de son dichlorhydrate >250° C avec décomposition (méthanol anhydre), à partir:
– de chlorométhyl-6 benzodioxine et de (pyridyl-2)-1 pipérazine, ou
– de chloro-2 pyridine et de (benzodioxinyl-6 méthyl)-1 pipérazine, ou
– de formyl-6 benzodioxine et de (pyridyl-2)-1 pipérazine.

3. (Benzodioxinyl-6 méthyl)-1 (thiazol-1,3 yl-2)-4 pipérazine, p.f. de son dichlorhydrate dihydrate >220° C (méthanol anhydre) à partir:
– de chlorométhyl-6 benzodioxine et de (thiazol-1,3 yl-2)-1 pipérazine, ou
– de chloro-2 thiazole-1,3 et de (benzodioxinyl-6 méthyl)-1 pipérazine, ou
– de formyl-6 benzodioxine et de (thiazol-1,3 yl-2)-1 pipérazine.

4. (Benzodioxinyl-6 méthyl)-1 (thiazol-1,3,4 yl-2)-4 pipérazine, p.f. de son dichlorhydrate >250° C avec décomposition (éthanol anhydre), à partir:
– de chlorométhyl-6 benzodioxine et de (thiadiazol-1,3,4 yl-2)-1 pipérazine, ou
– de chloro-2 thiadiazol-1,3,4 et de (benzodioxinyl-6 méthyl)-1 pipérazine, ou
– de formyl-6 benzodioxine et de (thiadiazol-1,3,4 yl-2)-1 pipérazine.

5. (Benzodioxinyl-6 méthyl)-1 (pyrazinyl-2)-4 pipérazine, p.f. de son dichlorhydrate >258° C avec décomposition (éthanol anhydre), à partir:
– de chlorométhyl-6 benzodioxine et de (pyrazinyl-2)-1 pipérazine, ou
– de chloro-2 pyrazine et de (benzodioxinyl-6 méthyl)-1 pipérazine, ou
– de formyl-6 benzodioxine et de (pyrazinyl-2)-1 pipérazine.

6. (Benzodioxinyl-6 méthyl)-1 (méthyl-4 pyridyl-2)-4 pipérazine, à partir:
– de chlorométhyl-6 benzodioxine et de (méthyl-4 pyridyl-2)-1 pipérazine, ou
– de méthyl-4 chloro-2 pyridine et de (benzodioxinyl-6 méthyl)-1 pipérazine, ou
– de formyl-6 benzodioxine et de (méthyl-4 pyridyl-2)-1 pipérazine.

7. (Benzodioxinyl-6 méthyl)-2 (méthoxy-6 pyridyl-2)-4 pipérazine, à partir:
– de chlorométhyl-6 benzodioxine et de (méthoxy-6 pyridyl-2)-1 pipérazine, ou
– de méthoxy-6 chloro-2 pyridine et de (benzodioxinyl-6 méthyl)-1 pipérazine, ou
– de formyl-6 benzodioxine et de (méthoxy-6 pyridyl-2)-1 pipérazine.

8. (Benzodioxinyl-6 méthyl)-2 (oxadiazol-1,2,4 yl-5)-4 pipérazine, à partir:
– de chlorométhyl-6 benzodioxine et d'(oxadiazol-1,2,4 yl-5)-1 pipérazine, ou
– de chloro-5 oxadiazole-1,2,4 et de (benzodioxinyl-6 méthyl)-1 pipérazine, ou
– de formyl-6 benzodioxine et d'(oxadiazol-1,2,4 yl-5)-1 pipérazine.

9. (Benzodioxinyl-6 méthyl)-1 (méthyl-4 triazol-1,2,4 yl-3)-4 pipérazine, à partir:
– de chlorométhyl-6 benzodioxine et de (méthyl-4 triazol-1,2,4 yl-3)-4 pipérazine, ou
– de chloro-3 méthyl-4 triazole-1,2,4 et de (benzodioxinyl-6 méthyl)-1 pipérazine, ou
– de formyl-6 benzodioxine et de (méthyl-4 triazol-1,2,4 yl-3)-1 pipérazine.

**Revendications** pour les Etats contractants BE, CH, DE, FR, GB, IT, LI, LU, NL, SE

1. Les dérivés de benzodioxine de formule générale:

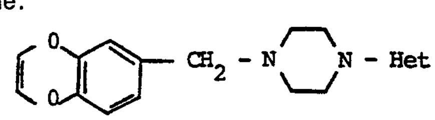 (I)

dans laquelle Het représente un radical pyridyle, pyrimidinyle, pyrazinyle, thiazolyle, thiadiazolyle, oxadiazolyle ou triazolyle éventuellement substitué par un radical méthyle ou méthoxy.

2. Les sels d'addition des composés selon la revendication 1 avec des acides appropriés.

3. Les sels selon la revendication 2 qui sont physiologiquement tolérables.

4. La (benzodioxinyl-6 méthyl)-1 (pyrimidinyl-2)-4 pipérazine et son dichlorhydrate.

5. La (benzodioxinyl-6 méthyl)-1 (pyridyl-2)-4 pipérazine et son dichlorhydrate.

6. La (benzodioxinyl-6 méthyl)-1 (thiazol-1,3 yl-2)-4 pipérazine et son dichlorhydrate.

7. La (benzodioxinyl-6 méthyl)-1 (thiadiazol-1,3,4 yl-2)-4 pipérazine et son dichlorhydrate.

8. Un procédé de préparation des composés de la revendication 1, caractérisé en ce que l'on condense un dérivé halogéné de formule générale:

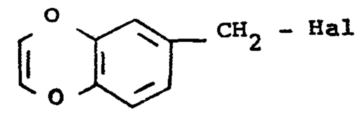 (II)

dans laquelle Hal est un atome de chlore ou de brome,
– avec une pipérazine N-monosubstituée de formule générale:

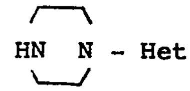 (III)

dans laquelle Het a la signification définie dans la revendication 1.

9. Un procédé de préparation des composés de la revendication 1, caractérisé en ce que l'on condense un dérivé halogéné de formule générale:

Het-Hal (IV)

dans laquelle Het et Hal ont les significations énoncées respectivement dans les revendications 1 et 8,
– avec une pipérazine N-monosubstituée de formule:

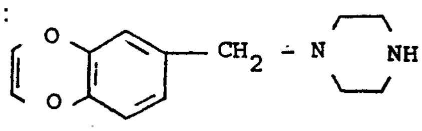 (V)

10. Un procédé de préparation des composés de la revendication 1, caractérisé en ce que l'on soumet un mélange de l'aldéhyde de formule:

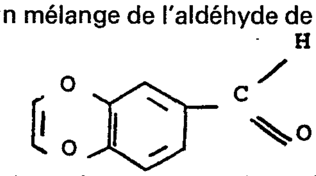 (VI)

et d'une pipérazine N-monosubstituée de formule générale (III) telle que définie dans la revendication 8, à une alkylation réductive à l'aide d'un cyanoborohydrure de formule:

$BH_3CN\ M$

dans laquelle M représente un métal alcalin.

11. Les compositions pharmaceutiques contenant comme principe actif au moins un composé selon les revendications 1 et 3 à 7 avec les supports pharmaceutiques appropriés.

12. Les compositions pharmaceutiques selon la revendication 11, présentées sous une forme convenant pour l'administration par voie orale, rectale ou parentérale, notamment dans le traitement des voies dopaminergiques.

**Revendication** pour l'Etat contractant: AT

Procédé de préparation des dérivés de benzodioxine de formule générale:

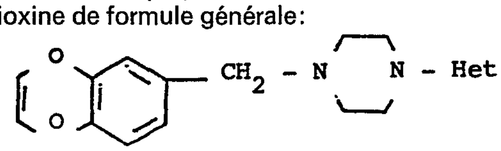 (I)

dans laquelle Het représente un radical pyridyle, pyrimidinyle, pyrazinyle, thiazolyle, thiadiazolyle, oxadiazolyle ou triazolyle éventuellement substitué par un radical méthyle ou méthoxy et de leurs sels d'addition avec des acides appropriés, caractérisé en ce que:

– soit l'on condense un dérivé halogéné de formule générale:

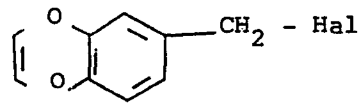

(II)

dans laquelle Hal est un atome de chlore ou de brome,

– avec une pipérazine N-monosubstituée de formule générale:

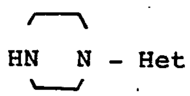

(III)

dans laquelle Het a la signification précédemment définie;

– soit l'on condense un dérivé halogéné de formule générale:

Het-Hal (IV)

dans laquelle Het et Hal ont les significations énoncées précédemment,

– avec une pipérazine N-monosubstituée de formule:

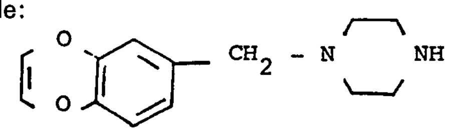

(V)

– soit l'on soumet un mélange de l'aldéhyde de formule:

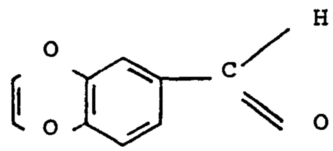

(VI)

et d'une pipérazine N-monosubstituée de formule générale (III) telle que définie ci-dessus, à une alkylation réductive à l'aide d'un cyanoborohydrure de formule:

$BH_3CN$ M

dans laquelle M représente un métal alcalin; et, si on le désire, on traite les dérivés (I) ainsi obtenus avec des acides appropriés pour donner les sels d'addition correspondants.

**Patentansprüche** für die Vertragsstaaten BE, CH, DE, FR, GB, IT, LI, LU, NL, SE

1. Benzodioxinderivate der allgemeinen Formel (I)

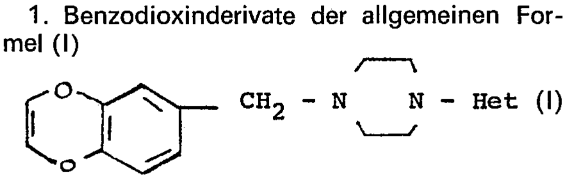

(I)

in der Het einen Pyridyl-, Pyrimidinyl-, Pyrazinyl-, Thiazolyl-, Thiadiazolyl-, Oxadiazolyl- oder Triazolylrest, der ggf. durch eine Methylgruppe oder eine Methoxygruppe substituiert ist, bedeutet.

2. Die Additionssalze der Verbindungen nach Anspruch 1 mit geeigneten Säuren.

3. Die Salze nach Anspruch 2, die physiologisch verträglich sind.

4. 1-(Benzodioxin-6-yl-methyl)-4-(pyrimidin-2-yl)-piperazin und dessen Dihydrochlorid.

5. 1-(Benzodioxin-6-yl-methyl)-4-(pyrid-2-yl)-piperazin und dessen Dihydrochlorid.

6. 1-(Benzodioxin-6-yl-methyl)-4-(1,3-thiazol-2-yl)-piperazin und dessen Dihydrochlorid.

7. 1-(Benzodioxin-6-yl-methyl)-4-(1,3,4-thiadiazol-2-yl)-piperazin und dessen Dihydrochlorid.

8. Verfahren zur Herstellung der Verbindungen nach Anspruch 1, dadurch gekennzeichnet, dass man ein Halogenderivat der allgemeinen Formel (II)

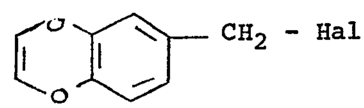

(II)

in der Hal für ein Chloratom oder ein Bromatom steht, mit einem N-monosubstituierten Piperazin der allgemeinen Formel (III)

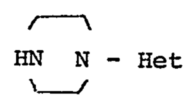

(III)

in der Het die in Anspruch 1 angegebene Bedeutung besitzt, kondensiert.

9. Verfahren zur Herstellung der Verbindungen nach Anspruch 1, dadurch gekennzeichnet, dass man ein Halogenderivat der allgemeinen Formel (IV)

Het-Hal (IV)

in der Het und Hal die in den Ansprüchen 1 bzw. 8 angegebenen Bedeutungen besitzen, mit einem N-monosubstituierten Piperazin der allgemeinen Formel (V)

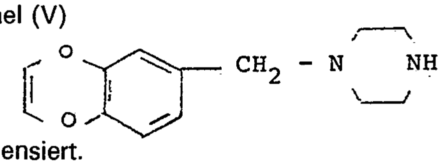

(V)

kondensiert.

10. Verfahren zur Herstellung der Verbindungen nach Anspruch 1, dadurch gekennzeichnet, dass man eine Mischung aus einem Aldehyd der Formel (VI)

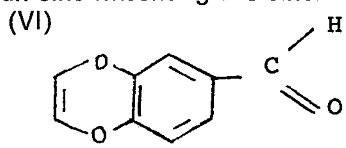

(VI)

und einem N-monosubstituierten Piperazin der allgemeinen Formel (III), wie sie in Anspruch 8 definiert ist, einer reduktiven Alkylierung mit einem Cyanoborhydrid der Formel

$BH_3CN$ M

in der M für ein Alkalimetall steht, unterwirft.

11. Pharmazeutische Zubereitungen enthaltend als Wirkstoff mindestens eine Verbindung nach einem der Ansprüche 1 und 3 bis 7 mit einem geeigneten pharmazeutischen Trägermaterial.

12. Pharmazeutische Zubereitungen nach Anspruch 11, dadurch gekennzeichnet, dass sie in

einer für die Verabreichung auf oralem, rektalem oder parenteralem Wege geeigneten Form vorliegen, insbesondere zur Behandlung von psychotropen Störungen, die mit Funktionsstörungen der dopaminergen Wege verbunden sind.

**Patentanspruch** für den Vertragsstaat: AT

Verfahren zur Herstellung von Benzodioxinderivaten der allgemeinen Formel (I)

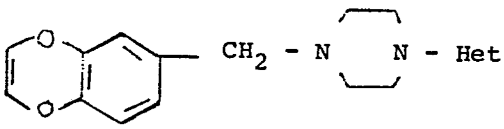
(I)

in der Het einen Pyridyl-, Pyrimidinyl-, Pyrazinyl-, Thiazolyl-, Thiadiazolyl-, Oxadiazolyl- oder Triazolylrest, der ggf. durch eine Methylgruppe oder eine Methoxygruppe substituiert ist, bedeutet, und ihrer Additionssalze mit geeigneten Säuren, dadurch gekennzeichnet, dass man entweder ein Halogenderivat der allgemeinen Formel (II)

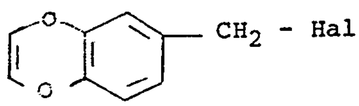
(II)

in der Hal ein Chloratom oder ein Bromatom bedeutet, mit einem N-monosubstituierten Piperazin der allgemeinen Formel (III)

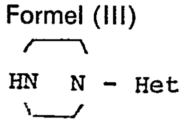
(III)

in der Het die oben angegebene Bedeutung besitzt, kondensiert; oder ein Halogenderivat der allgemeinen Formel (IV)

Het-Hal (IV)

in der Het und Hal die oben angegebenen Bedeutungen besitzen, mit einem N-monosubstituierten Piperazin der allgemeinen Formel (V)

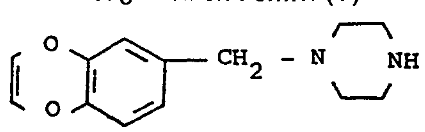
(V)

kondensiert; oder eine Mischung aus einem Aldehyd der allgemeinen Formel (VI)

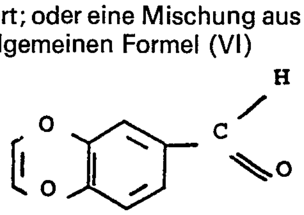
(VI)

und einem N-monosubstituierten Piperazin der oben definierten allgemeinen Formel (III), einer reduktiven Alkylierung mit einem Cyanoborhydrid der Formel

$BH_3CN$ M

in der M ein Alkalimetall darstellt, unterwirft; und gewünschtenfalls die in dieser Weise erhaltenen Derivate der allgemeinen Formel (I) mit geeigneten Säuren behandelt, um die entsprechenden Additionssalze zu bilden.

**Claims** for the Contracting States BE, CH, DE, FR, GB, IT, LI, LU, NL, SE

1. Benzodioxin derivatives of the general formula:

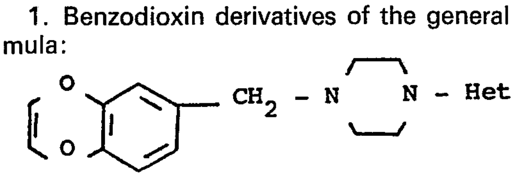
(I)

in which Het represents a pyridyl, pyrimidinyl, pyrazinyl, thiazolyl, thiadiazolyl, oxadiazolyl or triazolyl radical that is optionally substituted by a methyl or methoxy radical.

2. The addition salts of the compounds according to Claim 1 with suitable acids.

3. The salts according to Claim 2 that are physiologically tolerable.

4. 1-(benzodioxin-6-ylmethyl)-4-(pyrimidin-2-yl)-piperazine and the dihydrochloride thereof.

5. 1-(benzodioxin-6-ylmethyl)-4-(pyrid-2-yl)-piperazine and the dihydrochloride thereof.

6. 1-(benzodioxin-6-ylmethyl)-4-(1,3-thiazol-2-yl)-piperazine and the dihydrochloride thereof.

7. 1-(benzodioxin-6-ylmethyl)-4-(1,3,4-thiadiazol-2-yl)-piperazine and the dihydrochloride thereof.

8. A process for the preparation of the compounds of Claim 1, characterised in that a halogenated derivative of the general formula:

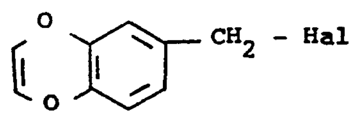
(II)

in which Hal is a chlorine or bromine atom, is condensed
– with an N-mono-substituted piperazine of the general formula:

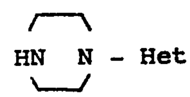
(III)

in which Het has the meaning defined in Claim 1.

9. A process for the preparation of the compounds of Claim 1, characterised in that a halogenated derivative of the general formula:

Het-Hal (IV)

in which Het and Hal have the meanings given in Claims 1 and 8, respectively, is condensed
– with an N-mono-substituted piperazine of the formula:

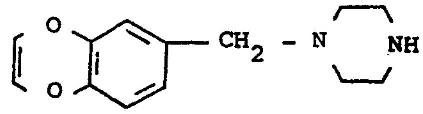
(V)

10. A process for the preparation of the compounds of Claim 1, characterised in that a mixture of an aldehyde of the formula:

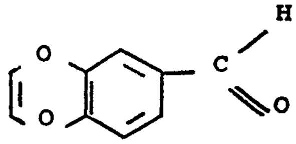
(VI)

and an N-mono-substituted piperazine of the general formula (III) as defined in Claim 8 is subjected to reductive alkylation using a cyanoborohydride of the formula:

$BH_3CN\ M$

in which M represents an alkali metal.

11. Pharmaceutical compositions containing as active ingredient at least one compound according to Claims 1 and 3 to 7 with suitable pharmaceutical carriers.

12. Pharmaceutical compositions according to Claim 11 in a form suitable for oral, rectal or parenteral administration, especially in the treatment of psychotropic disorders connected with a dysfunction of the dopaminergic pathways.

**Claim** for the Contracting State: AT

Process for the preparation of benzodioxin derivatives of the general formula:

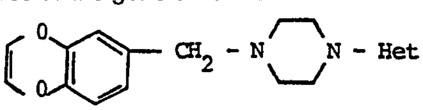 (I)

in which Het represents a pyridyl, pyrimidinyl, pyrazinyl, thiazolyl, thiadiazolyl, oxadiazolyl or triazolyl radical that is optionally substituted by a methyl or methoxy radical and the addition salts thereof with suitable acids, characterised in that:
– a halogenated derivative of the general formula:

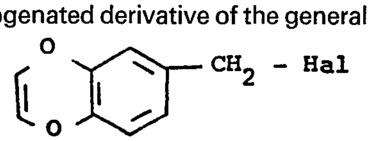 (II)

in which Hal is a chlorine or bromine atom, is condensed

– with an N-mono-substituted piperazine of the general formula:

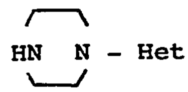 (III)

in which Het has the meaning defined above;
– or a halogenated derivative of the general formula:

Het-Hal (IV)

in which Het and Hal have the meanings given above, is condensed
– with an N-mono-substituted piperazine of the formula:

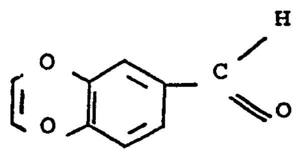 (V)

– or a mixture of an aldehyde of the formula:

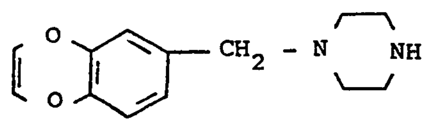 (VI)

and an N-mono-substituted piperazine of the general formula (III) as defined above is subjected to reductive alkylation using a cyanoborohydride of the formula:

$BH_3CN\ M$

in which M represents an alkali metal;
– and, if desired, the resulting derivatives (I) are treated with suitable acids to yield the corresponding addition salts.